# EUROPÄISCHE PATENTSCHRIFT

(11) **EP 2 581 061 B1**
(45) Veröffentlichungstag und Bekanntmachung des Hinweises auf die Patenterteilung: **19.04.2017**
(21) Anmeldenummer: 11193945.0
(22) Anmeldetag: 16.12.2011
(51) Int. Cl.: A61C 1/00, A61B 90/98

(54) **Kupplungsvorrichtung zur lösbaren Verbindung eines medizinischen, insbesondere dentalen, Instruments mit einer Antriebseinheit oder einem Versorgungsschlauch**
Coupling device for a releasable connection of a medical, in particular dental instrument with a drive unit or a supply hose
Dispositif d'accouplement pour la liaison amovible d'un instrument médical, notamment dentaire, avec une unité d'entraînement ou un tuyau d'alimentation

(30) Priorität: 10.10.2011 EP 11184479
(43) Veröffentlichungstag der Anmeldung: 17.04.2013
(73) Patentinhaber: W & H Dentalwerk Bürmoos GmbH, 5111 Bürmoos (AT)
(72) Erfinder: Mangelberger, Michael, 5113 St. Georgen (AT); Baier, Christof, 5111 Bürmoos (AT); Tannebaum, Wolfgang, 92637 Weiden (DE)

(56) Entgegenhaltungen:
- EP-A2- 2 327 370
- EP-A2- 2 359 756
- EP-B1- 1 392 193
- DE-A1-102009 008 698
- US-A1- 2006 142 744

## Beschreibung

Die vorliegende Erfindung bezieht sich auf eine Kupplungsvorrichtung zur lösbaren Verbindung eines medizinischen, insbesondere dentalen, Instruments mit einer Antriebseinheit oder einem Versorgungsschlauch nach dem Oberbegriff des Anspruchs 1.

Derartige Kupplungsvorrichtungen dienen zur Übertragung von Daten, Energie, Licht, einer Antriebsbewegung und/ oder eines Arbeitsmediums, insbesondere eines Fluids, zwischen zwei medizinischen, insbesondere dentalen, Vorrichtungen. Eine der Vorrichtungen ist hierbei vorzugsweise als medizinisches Instrument, insbesondere als dentales Hand- oder Winkelstück zum Anschluss medizinischer, insbesondere dentaler Werkzeuge, welche bevorzugt zur Bearbeitung von hartem oder weichem Gewebe oder zum Einbringen von Implantaten dienen, ausgebildet. Des Weiteren werden unter dem Begriff Instrumente insbesondere gerade, gebogene oder pistolenförmige Handstücke als auch Teile von Handstücken, Adapter und Lichtsonden verstanden. Bevorzugt werden die Hand- oder Winkelstücke mittels der Kupplungsvorrichtung, insbesondere mittels eines ersten Kupplungselements der Kupplungsvorrichtung, mit dem zweiten Kupplungselement, welches vorzugsweise an einer medizinischen, insbesondere dentalen, Antriebseinheit oder einem Versorgungsschlauch angeordnet ist, verbunden, um die medizinischen Werkzeuge durch einen in der Antriebseinheit angeordneten Elektromotor anzutreiben. Während des Betriebs der medizinischen Werkzeuge sind diese sowie die Hand- oder Winkelstücke mit Arbeitsmedien, wie zum Beispiel Sprayluft und/ oder Spraywasser zur Kühlung oder elektrischer Energie, zu versorgen. Diese Medien werden insbesondere von einer dentalen Einheit bereit gestellt und mittels einer Versorgungsleitung über die Kupplungsvorrichtung der Antriebseinheit oder dem Hand- oder Winkelstück zugeführt.

Zur Identifizierung der Instrumente, insbesondere der Hand- und Winkelstücke, um die Arbeitsmedien automatisch, abhängig von dem über den Versorgungsschlauch mit der Einheit verbundenen Instrument, zuzuführen, weisen diese oftmals eine Codierung auf. Diese Codierung, welche insbesondere Identifizierungsdaten wie z.B. eine Seriennummer enthält, wird üblicherweise von einer Elektronik der dentalen Einheit ausgelesen, um so die jeweiligen Betriebsparameter für die Arbeitsmedien aus einer Speichereinheit in der dentalen Einheit für den Betrieb des mit der Einheit gekoppelten Instruments auszuwählen.

Des Weiteren ist es bekannt, neben den Identifizierungsdaten auch die Betriebsparameter für den Betrieb des Instruments in dem medizinischen Instrument, insbesondere in einem darin angeordneten Datenspeicher, zu hinterlegen. Hierbei erkennt die Elektronik der dentalen Einheit mittels der Identifizierungsdaten das Instrument und stellt gleichzeitig mittels der in dem Speicher des Instruments hinterlegten Betriebsparameterdaten die Parameter für die Arbeitsmedien an der dentalen Einheit ein. Unter den Betriebsparameterdaten der Instrumente werden insbesondere Drehmomente, Drehzahlen, Übertragungsverhältnisse und Wirkungsgrade verstanden.

Ebenso ist es bekannt, den Datenspeicher beschreibbar bzw. überschreibbar auszugestalten. Neben den Identifizierungs- und Betriebsparameterdaten, welche auf dem Speicher hinterlegt sind, können zusätzlich weitere Daten, insbesondere Instandhaltungsdaten, wie z.B. Laufzeiten, Servicearbeiten, Pflege- oder Reinigungsdaten auf den Speicher geschrieben und/ oder auf diesem gesammelt werden.

Ein medizinisches Instrument mit einer derartigen Kupplungsvorrichtung mit einem ersten und zweiten Kupplungselement zum Versorgen des Instruments mit Medien sowie mit einem Speicher im Inneren des medizinischen Instruments ist aus der EP 1 392 193 B1 bekannt.

Dieses bekannte Instrument umfasst eine Außenhülse in der eine Kupplungsausnehmung eines ersten Kupplungselements zum Einsetzen eines Kupplungszapfens eines zweiten Kupplungselements einer Kupplungsvorrichtung zum Versorgen des Instruments mit Medien, insbesondere mit Wasser, Druckluft oder Licht, vorgesehen ist. In der Ausnehmung ist eine Arbeitswelle angeordnet, die mit einer Antriebswelle des anderen Kupplungselements kuppelbar ist, so dass die Arbeitswelle des Instruments bevorzugt in eine Drehbewegung versetzbar ist. Im Inneren des medizinischen Instruments ist ein Speicherelement für Identifikations- und/ oder Betriebsparameterdaten vorgesehen. Dieses Speicherelement ist in der die Kupplungsausnehmung umgebenden Hülsenwand des Instruments angeordnet und für das korrespondierende Kupplungselement von der Innenmantelfläche der Hülsenwand oder von der hinteren Ringstirnfläche der Hülsenwand her zugänglich. In einem weiteren Ausführungsbeispiel ist das Speicherelement in dem Kupplungszapfen der Kupplungsvorrichtung, welcher zum Versorgen des Instruments mit Medien dient, angeordnet und von der Mantelfläche des Kupplungszapfens her zugänglich oder in der Kupplungszapfenbasis hinter einer ringförmigen Stufenfläche angeordnet und von der ringförmigen Stufenfläche her zugänglich.

Die EP 2 359 756 A2 offenbart eine weitere Kupplungsvorrichtung zur Verbindung eines medizinischen Instruments mit einer Antriebseinheit.

Zur elektrischen Kontaktierung beider Bauteile weist die Antriebseinheit einen elektrischen Ausgang auf, welcher sich von der Rückseite der Antriebseinheit erstreckt und in eine Öffnung des Instruments einführbar ist. Der elektrische Ausgang weist bevorzugt zwei elektrische Kontakte auf, welche mit elektrischen Kontaktgliedern an dem Instrument verbindbar sind. Die Kontaktglieder sind hierzu in der Öffnung zur Aufnahme des elektrischen Ausgangs der Antriebseinheit angeordnet. Des Weiteren sind die elektrischen Kontaktglieder des Instruments mit einem Speicherelement elektrisch verbunden. Das Speicherelement ist hierbei in einer Ausnehmung im Inneren des medizinischen Instruments angeordnet.

Als nachteilig dieser Ausgestaltungen, insbesondere der Anordnung des Speicherelements im Inneren des medizinischen Instruments, insbesondere in der die Kupplungsausnehmung umgebenden Hülsenwand, im Kupplungszapfen selbst oder in der Kupplungszapfenbasis, erweist sich die eingeschränkte Zugänglichkeit zu dem Speicherelement. Bei diesen bekannten Anordnungen ist das Speicherelement jeweils von einer oder maximal zwei Seiten her zugänglich. Somit können Daten und/ oder Energie nur eingeschränkt auf das Speicherelement und von diesem auf eine Übertragungseinheit übertragen werden.

Im medizinischen, insbesondere dentalen Bereich, ist es des Weiteren üblich die Außenhülsen der Instrumente sowie die beiden Kupplungselemente der Kupplungsvorrichtungen, insbesondere die Hülsen der Ausnehmungen sowie die Kupplungszapfen aus einem metallischen Werkstoff zu fertigen. Insbesondere bei einer induktiven Energie- und/ oder Datenübertragung zwischen Speicher und Lesevorrichtung ist es erforderlich alle im Bereich und zwischen den beiden Elementen liegenden Bauteile aus nicht elektrisch leitendem Material auszubilden, um den Energie- und Datentransfer nicht durch die in den elektrisch leitenden Bauteilen erzeugten Gegenfelder zu gefährden. Somit besteht bei der im Stand der Technik bekannten Anordnung des Speicherelements im Inneren des Instruments die Gefahr, dass durch elektrisch leitende, insbesondere metallische Bauteile, wie z.B. der Außenhülse, der Hülse für die Kupplungsausnehmung, dem Kupplungszapfen oder der darin gelagerten Wellen selbst, Gegenfelder erzeugt werden, welche eine induktive Energie- und/ oder Datenübertragung zwischen Speicher und Lesevorrichtung gefährden.

Einen weiteren Nachteil der im Stand der Technik bekannten Ausführungsformen stellt die fehlende Möglichkeit dar, bereits bestehende Kupplungselemente, insbesondere medizinische Instrumente, zum Senden und/ oder Empfangen von Daten und/ oder Energie auszubilden. Durch das Anordnen des Speicherelements im Inneren des Instruments, insbesondere in der die Ausnehmung bildenden Kupplungshülse, ist ein Nachrüsten bestehender Instrumente mit einem Speicherelement nicht bzw. nur mit erheblichen Aufwand möglich.

Der vorliegenden Erfindung liegt daher die Aufgabe zugrunde eine Kupplungsvorrichtung zur lösbaren Verbindung eines medizinischen, insbesondere dentalen, Instruments mit einer Antriebseinheit oder einem Versorgungsschlauch zu schaffen, die es insbesondere ermöglicht Daten und/ oder Energie von dem einen Kupplungselement auf das andere Kupplungselement der Kupplungsvorrichtung sicher zu übertragen, als auch bereits bestehende Kupplungselemente, insbesondere medizinische Instrumente, zum Senden und/ oder Empfangen von Daten und/ oder Energie auszubilden.

Gemäß einem Ausführungsbeispiel einer Kupplungsvorrichtung zur lösbaren Verbindung eines medizinischen, insbesondere dentalen, Instruments mit einer Antriebseinheit oder einem Versorgungsschlauch zur Übertragung von Daten und/ oder Energie und gegebenenfalls einer Antriebsbewegung und/ oder eines Arbeitsmediums, insbesondere eines Fluids, zwischen der Antriebseinheit oder dem Versorgungsschlauch und dem Instrument, weist diese ein erstes und zweites Kupplungselement auf, wobei eines der beiden Kupplungselemente als Kupplungsausnehmung ausgebildet ist, in die ein Kupplungsvorsprung des anderen Kupplungselements einsetzbar ist, wobei an einem der beiden Kupplungselemente ein Positionierungselement angeordnet ist, welches in eine Vertiefung an dem anderen Kupplungselement einführbar ist, um beide Kupplungselemente in einer definierten Winkelposition um deren gemeinsame Achse zueinander zu positionieren, und wobei das Positionierungselement eine Speichereinheit zur Speicherung von, vorzugsweise instrumentenbezogenen, Daten und die Vertiefung eine Übertragungseinheit aufweist, wobei die Speichereinheit derart in dem Positionierungselement angeordnet ist, dass diese in die Vertiefung des anderen Kupplungselements einführbar ist, so dass Daten von der Speichereinheit des einen Kupplungselements mittels der Übertragungseinheit auf das andere Kupplungselement der Kupplungsvorrichtung übertragbar sind.

Gemäß einem ersten Ausführungsbeispiel der Kupplungsvorrichtung weist das Positionierungselement des einen Kupplungselements die Speichereinheit auf, wobei zur Übertragung der, insbesondere instrumentenbezogenen, Daten auf das andere Kupplungselement das Positionierungselement zumindest einen mit der Speichereinheit verbundenen elektrischen Kontakt aufweist, welcher mit einem elektrischen Kontakt des anderen Kupplungselements, insbesondere der Übertragungseinheit, welche in der Vertiefung zur Aufnahme des Positionierungselements angeordnet ist, verbindbar ist, so dass die Daten und/ oder Energie leitungsgebunden zwischen den beiden Kupplungselementen übertragbar sind. Der zumindest eine elektrische Kontakt der Speichereinheit ist hierzu insbesondere in der Stirnfläche des Positionierungselements und der zumindest eine elektrische Kontakt der Übertragungseinheit in einer Basisfläche der Vertiefung angeordnet und so für den jeweils anderen Kontakt zugänglich.

Das Positionierungselement selbst ist vorzugsweise aus einem magnetisch nicht leitenden und elektrisch nicht leitenden Material, insbesondere aus einem Kunststoff, Glas oder Keramik, gefertigt und federnd an dem einen Kupplungselement der Kupplungsvorrichtung gelagert, so dass dieses im Wesentlichen parallel zur Drehachse der Kupplungsvorrichtung verschiebbar ist. Des Weiteren ist das Element bevorzugt lösbar von dem Kupplungselement ausgebildet ist, so dass dieses austauschbar ist.

Die Speichereinheit selbst umfasst bevorzugt einen elektronisch programmierbaren Speicher, der entweder als lesbarer Speicher oder als lesbarer und beschreibbarer Speicher ausgebildet ist. Die Übertragungseinheit ist bevorzugt durch eine Empfangseinheit mit einem Reader zur Verarbeitung und/ oder zur Aufbereitung der übermittelten Daten ausgebildet.

Gemäß einem zweiten Ausführungsbeispiel der Kupplungsvorrichtung weist das Positionierungselement mit der Speichereinheit des einen Kupplungselements eine Lese- und/ oder Schreibvorrichtung, insbesondere eine erste Spule auf, wobei eine zweite Spule zur Daten und/ oder Energieübertragung in der Vertiefung des anderen Kupplungselements angeordnet ist. Beide Spulen sind hierzu an einer oder an mehreren Seitenwänden des Positionierungselements oder der Vertiefung, an der Stirnfläche des Positionierungselements oder an der Basisfläche der Vertiefung angebracht.

Die zumindest eine erste Spule der Speichereinheit sowie das Speicherelement sind vorzugsweise übereinander, alternativ nebeneinander auf einer gemeinsamen Trägerplatine, in einem eigenen Gehäuse angeordnet und bilden zusammen eine RFID-Einheit, welche wiederum in dem Positionierungselement, vorzugsweise mittels eines Spritzgussverfahrens, eingehaust ist.

Die Spulen der Speichereinheit und der Übertragungseinheit sind vorzugsweise von einem weichmagnetischen Material, insbesondere Ferrit, Nickel-Zink oder Mangan-Zink, zum Leiten und/ oder Ausrichten der Feldlinien des magnetischen Felds in Richtung der ersten oder zweiten Spule und/ oder von einem nicht elektrisch leitfähigem Material, insbesondere Kunststoff, Silikon, Harz, Keramik, Klebstoff, Lack oder Glas, zur Vermeidung der Schwächung des magnetischen Felds durch selbsterzeugte und/ oder empfangene Fremdfelder umgeben.

Gemäß einem dritten Ausführungsbeispiel der Kupplungsvorrichtung weist das erste und zweite Kupplungselement neben der Speichereinheit zur Speicherung von Daten und der Übertragungseinheit weitere elektrische Kontakte und/ oder Lese- und/ oder Schreibvorrichtungen zur leitungsgebundenen und/ oder drahtlosen Übertragung von Daten, Signalen und/ oder Energie von dem einen Kupplungselement zu dem anderen Kupplungselement der Kupplungsvorrichtung auf.

Gemäß allen vorstehenden Ausführungsbeispielen sind das Positionierungselement sowie die Vertiefung zur drehfesten Verbindung beider Kupplungselemente der Kupplungsvorrichtung ausgebildet. Insbesondere wenn das Positionierungselement und die Vertiefung ineinander greifen, ist kein Verdrehen beider Kupplungselemente um deren gemeinsame Drehachse möglich.

Die vorliegende Kupplungsvorrichtung zeichnet sich durch folgende Vorteile aus.

Die erfindungsgemäße Kupplungsvorrichtung ermöglicht Daten und/ oder Energie von dem einen Kupplungselement auf das andere Kupplungselement der Kupplungsvorrichtung sicher zu übertragen. Durch die Anordnung der Speichereinheit zur Speicherung von, vorzugsweise instrumentenbezogenen, Daten in dem Positionierungselement der Kupplungsvorrichtung ist dieses außerhalb der metallischen Bauteile der Kupplungsvorrichtung, insbesondere außerhalb der Außenhülse des mit dem Kupplungselement verbundenen Instruments, der Hülse für die Kupplungsausnehmung, dem Kupplungszapfen und der darin gelagerten Wellen, angeordnet. Die Gefahr der Erzeugung von Gegenfeldern bei einer induktiven Energie- und/ oder Datenübertragung zwischen Speicher und Lesevorrichtung wird somit vermieden.

Durch die bevorzugte Anordnung der Übertragungseinheit in einer Vertiefung an dem anderen Kupplungselement der Kupplungsvorrichtung ist dieses mechanisch geschützt gegen Fremdeinwirkung angeordnet.

Des Weiteren ist die Speichereinheit für die Übertragungseinheit von mehreren Seiten her zugänglich bzw. beschreib und/ oder auslesbar. Dies ermöglicht ebenfalls eine sichere Daten- und Energieübertragung.

Einen weiteren Vorteil der Erfindung bildet die Möglichkeit bereits bestehende Kupplungselemente, insbesondere medizinische Instrumente, zum Senden und/ oder Empfangen von Daten und/ oder Energie auszubilden. Dadurch, dass die Positionierungselemente bei bestehenden medizinischen Instrumenten lösbar ausgebildet sind, ist es möglich diese durch Positionierungselemente mit jeweils einer Speichereinheit mit, insbesondere instrumentenbezogende, Daten nachzurüsten. Trotz der Umrüstung bleiben die Instrumente bzw. Kupplungselemente weiterhin, ohne funktionelle Einschränkung, kompatibel mit den bestehenden Antriebseinheiten bzw. mit deren Kupplungselementen.

Im Rahmen der Erfindung versteht es sich selbstverständlich, dass die oben beschriebene Kupplungsvorrichtung nicht auf die Verwendung bei einem medizinischen, insbesondere dentalen, Instrument und einer Antriebseinheit beschränkt ist. Die Kupplungsvorrichtung kann vielmehr zur lösbaren Verbindung zweier medizinischer, insbesondere dentaler, Vorrichtungen verwendet werden. Vorzugsweise ist zumindest eine der medizinischen, insbesondere dentalen, Vorrichtungen als Reinigungs- und/ oder Pflegegerät, als Antriebseinheit, Versorgungsschlauch oder als Instrument mit einer Antriebsvorrichtung für ein Werkzeug ausgebildet.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel der Kupplungsvorrichtung mit einem ersten Kupplungselement an einem medizinischen, insbesondere dentalen, Instrument und einem zweiten Kupplungselement an einer Antriebseinheit,
Figur 2 eine Schnittdarstellung des ersten Ausführungsbeispiels der Kupplungsvorrichtung aus Figur 1 mit einer Speichereinheit aufweisend ein Speicherelement und einen elektrischen Kontakt in dem Positionierungselement des einen Kupplungselements und einem weiteren elektrischen Kontakt als Übertragungseinheit in der Vertiefung zur Aufnahme des Positionierungselements in dem anderen Kupplungselement, in einem teilweise montierten Zustand beider Kupplungselemente,
Figur 3 ebenfalls in Schnittdarstellung ein zweites Ausführungsbeispiel der Kupplungsvorrichtung mit einer Lese- und/ oder Schreibvorrichtung, insbesondere einer ersten und zweiten Spule, in dem Positionierungselement und in der Vertiefung des ersten und zweiten Kupplungselements,
Figur 4 ein drittes Ausführungsbeispiel der Kupplungsvorrichtung mit einer ersten Spule und einem Speicherelement angeordnet in einem eigenen Gehäuse in dem Positionierungselement,
Figur 5 ein viertes Ausführungsbeispiel der Kupplungsvorrichtung, wobei die erste und zweite Spule der beiden Kupplungselemente von einem weichmagnetischen Material und einem nicht elektrisch leitfähigem Material umgeben sind,
Figur 6 ein fünftes Ausführungsbeispiel der Kupplungsvorrichtung, wobei die Kupplungselemente jeweils eine weitere Lese- und/ oder Schreibvorrichtung zur drahtlosen Übertragung von Daten, Signalen und/ oder Energie von dem einen Kupplungselement zu dem anderen Kopplungselement aufweist;

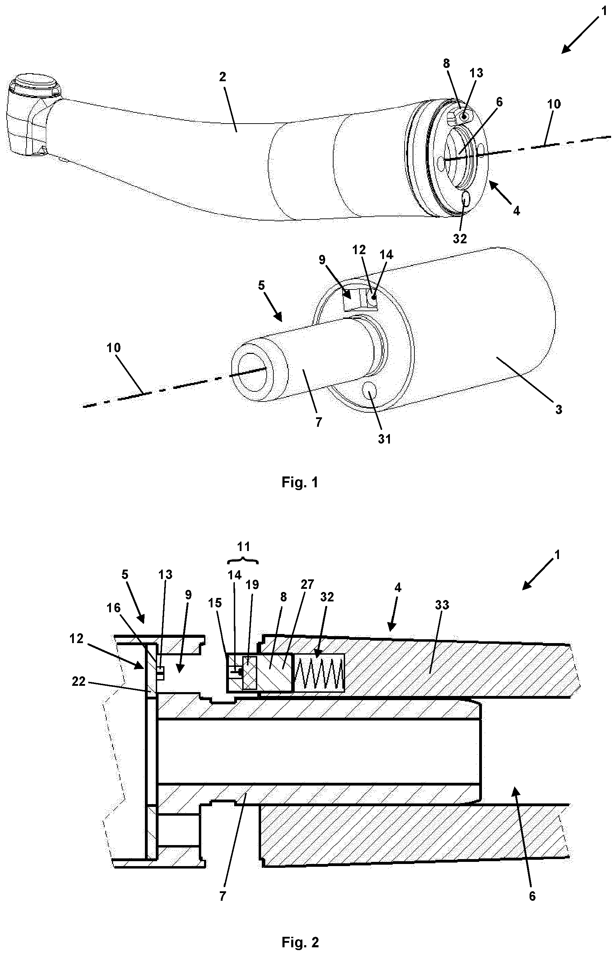

In Figur 1 ist eine Kupplungsvorrichtungen 1 zur lösbaren Verbindung eines medizinischen, insbesondere dentalen, Instruments 2 mit einer Antriebseinheit 3 zur Übertragung von Daten und/ oder Energie, einer Antriebsbewegung und eines Arbeitsmediums, insbesondere eines Fluids, zwischen der Antriebseinheit 3 und dem Instrument 2 abgebildet. Die Kupplungsvorrichtung 1 weist hierzu ein erstes Kupplungselement 4 in Form einer Kupplungsausnehmung 6 an dem medizinischen Instrument 2 und ein zweites Kupplungselement 5, welches als Kupplungsvorsprung 7 ausgestaltet ist, an der Antriebseinheit 3 auf.

Um beide Kupplungselemente 4, 5 in einer definierten Winkelposition um deren gemeinsame Achse 10, um welche sie bis zu einem gekoppelten Zustand beider Kupplungselemente 4, 5 frei drehbar sind, zueinander zu positionieren, um eine Medienleitung 31 der Antriebseinheit 3, insbesondere für Licht oder zumindest ein Fluid, zu der korrespondierenden Medienleitung 32 an dem medizinischen Instrument 2 auszurichten, weist die Kupplungsvorrichtung 1, insbesondere das erste Kupplungselement 4 mit der Kupplungsausnehmung 6 an dem Instrument 2, ein Positionierungselement 8 auf. Dieses erstreckt sich vorzugsweise von der Kupplungsfläche des ersten Kupplungselements 4 in entgegengesetzter Richtung zur Kupplungsausnehmung 6. Das Positionierungselement 8 ist vorzugsweise als Nase, Fortsatz oder Vorsprung ausgebildet und in eine Vertiefung 9 an dem anderen Kupplungselement 5 einführbar. Das Positionierungselement 8 und die Vertiefung 9 sind insbesondere zur drehfesten Verbindung beider Kupplungselemente 4, 5 ausgebildet.

Zur Identifizierung des medizinischen Instruments 2, um die Arbeitsmedien automatisch, abhängig von dem über die Antriebseinheit 3 mit einer dentalen Einheit verbundenen Instrument 2, zuzuführen, weist das Positionierungselement 8 des ersten Kupplungselements 4 eine Speichereinheit zur Speicherung von, vorzugsweise instrumentenbezogenen, Daten auf. Neben der Speichereinheit ist in diesem Ausführungsbeispiel an oder in dem Element 8 zumindest ein elektrischer Kontakt 13 angeordnet, welcher mit einem elektrischen Kontakt 14 einer Übertragungseinheit 12 in der Vertiefung 9 an dem anderen Kupplungselement 5 verbindbar ist, so dass die, insbesondere instrumentenbezogenen, Daten leitungsgebunden übertragbar sind.

Figur 2 zeigt die Kupplungsvorrichtung 1 aus Figur 1 in einer Schnittdarstellung in einem teilweise verbundenen Zustand beider Kupplungselemente 4, 5. Hierbei ist der Kupplungsvorsprung 7 des zweiten Kupplungselements 5 in der Kupplungsausnehmung 6 des ersten Kupplungselements 4, vorzugsweise des dentalen Instruments 2, aufgenommen. Das Positionierungselement 8 mit der Speichereinheit 11 des ersten Kupplungselements 4 ist zumindest teilweise in einer Ausnehmung in dem hülsenförmigen Kupplungsteil 33, insbesondere verschiebbar, gelagert und mittels einer Feder 32 relativ zur Vertiefung 9 an dem anderen Kupplungsteil 5 vorgespannt. Das Speicherelement 19 sowie der elektrische Kontakt 14 der Speichereinheit 11 selbst sind jedoch außerhalb des hülsenförmigen Kupplungsteils 33 in dem Positionierungselement 8 angeordnet, so dass diese von mehreren Seiten für das andere Kupplungselement 5 zugänglich sind. Das Gehäuse 27 des Positionierungselement 8, insbesondere jener Bereich, der die Speichereinheit 11 umgibt, umfasst bevorzugt ein magnetisch nicht leitendes und elektrisch nicht leitendes Material, insbesondere Kunststoff, Glas oder Keramik.

Die Übertragung der Daten und/ oder Energie zwischen der Speichereinheit 11, insbesondere zwischen dessen Speicherelement 19, und der Übertragungseinheit 12, welche bevorzugt in der Vertiefung 9 des anderen Kupplungselements 5 angeordnet ist, erfolgt in diesem Ausführungsbeispiel leitungsgebunden. Die elektrischen Kontakte 13, 14 sind jeweils an der Stirnfläche 15 des Positionierungselements 8 und an der Basisfläche 16 der Vertiefung 9 angebracht und/ oder für den korrespondierenden Kontakt 13, 14 zugänglich.

Der elektrische Kontakt 13 in der Vertiefung 9 zur Aufnahme des Positionierungselements 8 ist bevorzugt auf einer Trägerplatine 22 angeordnet. Die Platine 22 selbst ist vorzugsweise ringförmig ausgebildet; durch deren mittlere Öffnung erstreckt sich der Kupplungsvorsprung 7. Neben der Lese- und/ oder Schreibvorrichtung, insbesondere des elektrischen Kontakts 13, können auf der Platine 22 weitere elektronische Bauteile, insbesondere zur Daten und/ oder Energieübertragung, angeordnet sein. Vorzugsweise ist ein Leuchtmittel, insbesondere eine Leuchtdiode, auf der Platine 22 aufgelötet, wobei die Medienleitung 31 als Fenster der Diode ausgebildet ist, um Licht von der Antriebseinheit 3 auf das Instrument 2 zu übertragen. Die elektronischen Bauteile, die Übertragungseinheit 12 sowie die Platine 22 sind vorzugsweise in einem magnetisch nicht leitenden und elektrisch nicht leitenden Material, insbesondere Kunststoff, Glas oder Keramik eingehaust. Diese gekapselte Einheit umgibt den Kupplungsvorsprung 7, welcher sich von einer Kupplungsbasis durch die ringförmige Einheit erstreckt.

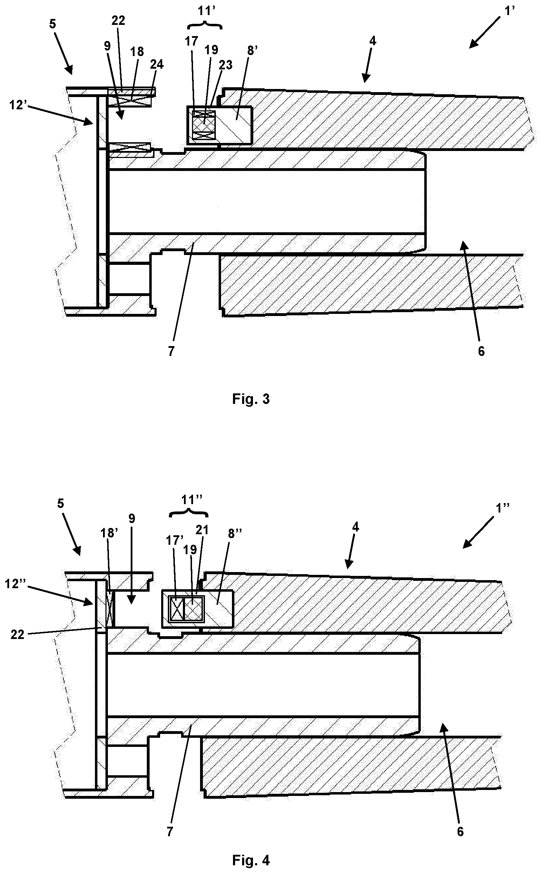

Figur 3 zeigt ebenfalls in Schnittdarstellung ein zweites Ausführungsbeispiel der Kupplungsvorrichtung 1' mit einer Lese- und/ oder Schreibvorrichtung in dem ersten und zweiten Kupplungselement 4, 5, insbesondere mit einer ersten Spule 17 in dem Positionierungselement 8' und einer zweiten Spule 18 in der Vertiefung 9. Das Positionierungselement 8' ist in diesem Beispiel fest mit dem hülsenförmigen Kupplungsteil, welcher die Kupplungsausnehmung 6 umgibt verbunden. Selbstverständlich ist es jedoch auch bei diesem sowie bei allen weiteren Ausführungsbeispielen möglich das Positionierungselement 8' mittels einer Feder relativ zur Vertiefung 9 an dem anderen Kupplungsteil 5 vorzuspannen. Auch hier ist die Speichereinheit 11' mit dem Speicherelement 19 und der Spule 17 außerhalb des hülsenförmigen Kupplungsteils in dem Positionierungselement 8' des ersten Kupplungselements 4 angeordnet. Die Spule 17 ist bevorzugt auf dem Speicherelement 19, insbesondere zwischen Speicherelement 19 und der Mantelfläche 23 des Positionierungselements 8', angeordnet. Die Spule 18 der Übertragungseinheit 12' des anderen Kupplungselements 5 mit dem Kupplungsvorsprung 7 ist auf einer, bevorzugt zumindest teilweise hülsenförmig ausgebildeten, Trägerplatine 22, welche in der Mantelfläche 24 der Vertiefung 9 angeordnet ist, aufgebracht. Beide Spulen 17, 18 sind somit zur induktiven Kopplung in einem montiertem Zustand der Kupplungsvorrichtung 1' ineinander angeordnet. Diese induktive Kopplung dient sowohl der Energieversorgung der Speichereinheit 11' als auch dem Datenaustausch. Bevorzugt erfolgt der Datenaustausch zwischen den beiden Spulen 17, 18 im Hochfrequenz- oder Radiofrequenzbereich, wobei die Speichereinheit 11' als RFID-Einheit, insbesondere als RFID-Etikett oder Transponder, bestehend aus einem RFID-Chip 19 und einer Spule 17 ausgebildet ist.

Die Figur 4 zeigt ein drittes Ausführungsbeispiel der Kupplungsvorrichtung 1" mit einem ersten und zweiten Kupplungselement 4, 5, aufweisend eine Speichereinheit 11" mit einer ersten Spule 17' und einem Speicherelement 19, welche in einem gemeinsamen Gehäuse 21 in dem Positionierungselement 8" angeordnet sind. Das Gehäuse 21 sowie die darin angeordneten Bauteile, insbesondere Speicher 19 und Spule 17', bilden eine RFID-Einheit, welche in dem Positionierungselement 8" vorzugsweise mittels eines Spritzgussverfahrens eingehaust ist. Alternativ kann der Speicher 19 und die Spule 17' in einem Glas-, Kunststoff-, Keramik-, oder Metallgehäuse 21 angeordnet sein. Die zweite Spule 18' der Übertragungseinheit 12" ist im Bereich der Basisfläche der Vertiefung 9 zur Aufnahme des Positionierungselements 8" auf einer Trägerplatine 22 angeordnet. Beide Spulen 17', 18' sind somit in einem gekoppelten Zustand beider Kupplungselemente 4, 5, insbesondere bei einer Anordnung des Kupplungsvorsprung 7 in der Kupplungsausnehmung 6, direkt nebeneinander bzw. gegenüberliegend angeordnet. Hierdurch wird ein besonders zuverlässiger Daten- und/ oder Energieaustausch erzielt.

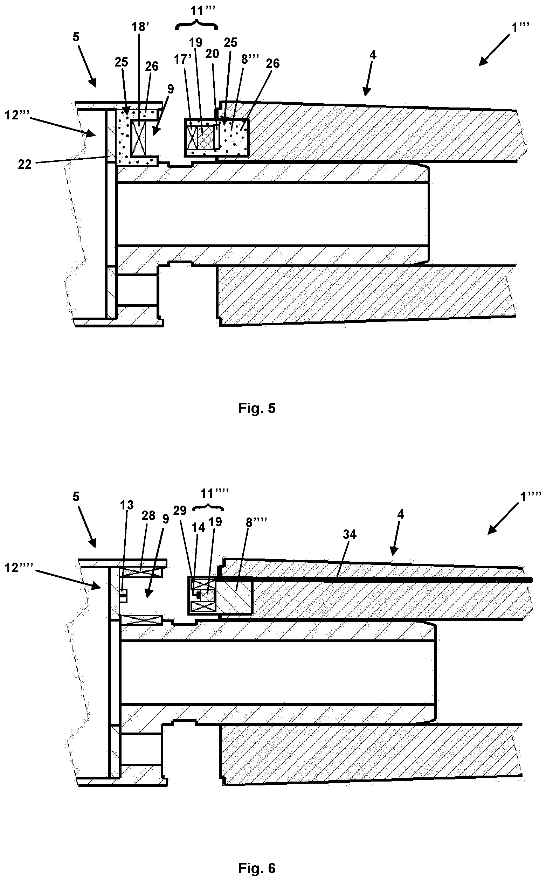

In Figur 5 ist ein viertes Ausführungsbeispiel der Kupplungsvorrichtung 1"' abgebildet, wobei die erste und zweite Spule 17', 18' der beiden Kupplungselemente 4, 5 von einem weichmagnetischen Material 25 und einem nicht elektrisch leitfähigem Material 26 umgeben sind. Die Spule 17' in dem Positionierungselement 8"' ist auf dem Speicherelement 19 positioniert, welches wiederum auf einer Trägerplatine 20 angeordnet ist. Die Trägerplatine 20 umfasst bevorzugt ein elektrisch isolierendes Material, zum Beispiel Kunststoff, Glas oder Keramik. Die Spule 18' der Übertragungseinheit 12"' des anderen Kupplungselements 5 ist ebenfalls auf einer derartigen Trägerplatine 22 angeordnet. Um einen besonders guten und störungsfreien Energie- und Datentransfer zu ermöglichen, ist gemäß einem bevorzugten Ausführungsbeispiel die Spule 18' der in der Vertiefung 9 angeordneten Übertragungseinheit 12"' sowie die Speichereinheit 11"' in dem Positionierungselement 8"' von einem weichmagnetischen Material 25, insbesondere Ferrit, Nickel-Zink oder Mangan-Zink, zum Leiten und/ oder Ausrichten der Feldlinien des magnetischen Felds in Richtung der ersten oder zweiten Spule 17', 18' und/ oder von einem nicht elektrisch leitfähigem Material 26, insbesondere Kunststoff, Silikon, Harz, Keramik, Klebstoff, Lack oder Glas, zur Vermeidung der Schwächung des magnetischen Felds durch selbsterzeugte und/ oder empfangene Fremdfelder umgeben. Bevorzugt umschließt das elektrisch nicht leitende Material 26 die Spule 18' und die Speichereinheit 11"' an allen Seiten. In diesem Ausführungsbeispiel weisen beide Bauteile 18', 11"' beide Materialien 25, 26 an zumindest drei Seiten auf, wobei das weichmagnetische Material 25 hierbei in Partikelform ausgebildet und jeweils in dem anderen Material 26 eingebettet ist.

Figur 6 zeigt ein fünftes Ausführungsbeispiel der Kupplungsvorrichtung 1"', wobei die Kupplungselemente 4, 5 jeweils eine weitere Lese- und/ oder Schreibvorrichtung 28, 29 zur drahtlosen Übertragung von Daten, Signalen und/ oder Energie von dem einen Kupplungselement zu dem anderen Kopplungselement aufweisen. Die Lese- und/ oder Schreibvorrichtung 28, 29 ist hierbei ebenfalls, neben den elektrischen Kontakten 13, 14 zum Daten und/ oder Energieaustausch zwischen dem Speicherelement 19 der Speichereinheit 11"' und der Übertragungseinheit 12"", in dem Positionierungselement 8"" und in der Vertiefung 9 der Übertragungseinheit 12"" angeordnet.

Wie bereits in den Figuren 1 und 2 gezeigt, sind die elektrischen Kontakte 13, 14 jeweils an der Stirnfläche des Positionierungselements 8"" und an der Basisfläche der Vertiefung 9 angebracht und für den korrespondierenden Kontakt 13, 14 zugänglich. Die Spulen 28, 29 zur zusätzlichen Übertragung von Daten, Signalen und/ oder Energie sind bevorzugt in einem montierten Zustand der Kupplungsvorrichtung 1"" ineinander angeordnet. Hierzu ist die Spule 29 bevorzugt zwischen dem Speicherelement 19 und der Mantelfläche des Positionierungselements 8"" und die Spule 28 der Übertragungseinheit 12"" des anderen Kupplungselements 5 bevorzugt in der Mantelfläche der Vertiefung 9 angeordnet.

Von der Spule 29 erstreckt sich vorzugsweise eine elektrische Leitung 34 zu einer elektronischen Komponente in dem medizinischen, insbesondere dentalen, Instrument, welches bevorzugt mit dem Kupplungselement 4 verbunden ist. Hierbei dienen die Spulen 28, 29 zur Energieübertragung, vorzugsweise für eine Lichtquelle in dem Instrument, und/ oder zur Übertragung von Daten, insbesondere Messdaten, wie. z.B. Sensordaten für Drehmomente, Temperaturen, und/ oder von Signalen, wie z.B. Wurzelkanalpositionssignale, von der elektronischen Komponente, wie z.B. einem Sensor oder einer Auswerteeinheit, zu einer dentalen Einheit.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Kupplungsvorrichtung (1, 1', 1", 1"', 1"") zur lösbaren Verbindung eines medizinischen, insbesondere dentalen, Instruments (2) mit einer Antriebseinheit (3) oder einem Versorgungsschlauch zur Übertragung von Daten und/ oder Energie und gegebenenfalls einer Antriebsbewegung und/ oder eines Arbeitsmediums, insbesondere eines Fluids, zwischen der Antriebseinheit (3) oder dem Versorgungsschlauch und dem Instrument (2), aufweisend ein erstes und zweites Kupplungselement (4, 5), wobei eines der beiden Kupplungselemente (4, 5) als Kupplungsausnehmung (6) ausgebildet ist, in die ein Kupplungsvorsprung (7) des anderen Kupplungselements (4, 5) einsetzbar ist, und wobei an einem der beiden Kupplungselemente (4, 5) ein Positionierungselement (8, 8', 8", 8"', 8"") angeordnet ist, welches in eine Vertiefung (9) an dem anderen Kupplungselement (4, 5) einführbar ist, um beide Kupplungselemente (4, 5) in einer definierten Winkelposition um deren gemeinsame Achse (10) zueinander zu positionieren, **dadurch gekennzeichnet, dass** das Positionierungselement (8, 8', 8", 8"', 8"") eine Speichereinheit (11, 11', 11", 11"', 11"") zur Speicherung von, vorzugsweise instrumentenbezogenen, Daten und die Vertiefung (9) eine Übertragungseinheit (12, 12', 12", 12"', 12"") aufweist, wobei die Speichereinheit (11, 11', 11", 11"', 11"") derart in dem Positionierungselement (8, 8', 8", 8"', 8"") angeordnet ist, dass diese in die Vertiefung (9) des anderen Kupplungselements (4, 5) einführbar ist, um Daten von der Speichereinheit (11, 11', 11", 11"', 11"") des einen Kupplungselements (4, 5) mittels der Übertragungseinheit (12, 12', 12", 12"', 12"") auf das andere Kupplungselement (4, 5) der Kupplungsvorrichtung (1, 1', 1", 1"', 1"") zu übertragen.

2. Kupplungsvorrichtung (1, 1"") nach Anspruch 1, **dadurch gekennzeichnet, dass** die Speichereinheit (11, 11"") zumindest einen elektrischen Kontakt (14) aufweist, welcher mit einem elektrischen Kontakt (13) der Übertragungseinheit (12, 12"") verbindbar ist, so dass die, insbesondere instrumentenbezogenen, Daten leitungsgebunden übertragbar sind.

3. Kupplungsvorrichtung (1, 1"") nach Anspruch 2, **dadurch gekennzeichnet, dass** ein elektrischer Kontakt (14), insbesondere jener der Speichereinheit (11, 11""), in einer Stirnfläche (15) des Positionierungselements (8, 8"") und ein elektrischer Kontakt (13), insbesondere jener der Übertragungseinheit (12, 12""), in einer Basisfläche (16) der Vertiefung (9) angeordnet ist.

4. Kupplungsvorrichtung (1', 1", 1"') nach Anspruch 1, **dadurch gekennzeichnet, dass** die Speichereinheit (11', 11", 11"') und die Übertragungseinheit (12', 12", 12"') jeweils eine Lese- und/ oder Schreibvorrichtung (17, 17'; 18, 18'), insbesondere eine erste und zweite Spule, zum Auslesen und/ oder Beschreiben des Speicherelements (19) mit insbesondere instrumentenbezogenen Daten umfasst.

5. Kupplungsvorrichtung (1"') nach Anspruch 4, **dadurch gekennzeichnet, dass** die zumindest eine erste Spule (17') der Speichereinheit (11"') und ein Speicherelement (19) der Speichereinheit (11"'), insbesondere übereinander oder nebeneinander, auf einer gemeinsamen Trägerplatine (20) in dem Positionierungselement (8"') angeordnet sind.

6. Kupplungsvorrichtung (1") nach Anspruch 4 oder 5, **dadurch gekennzeichnet, dass** die zumindest eine erste Spule (17') der Speichereinheit (11") und das Speicherelement (19) in einem gemeinsamen Gehäuse (21) in dem Positionierungselement (8") angeordnet sind.

7. Kupplungsvorrichtung (1', 1", 1"') nach einem der Ansprüche 4 bis 6, **dadurch gekennzeichnet, dass** die zumindest eine Spule (17, 17') des Positionierungselements (8', 8", 8"') an einer oder an mehreren Seitenwänden (23) und/ oder an der Stirnfläche (15) des Positionierungselements (8', 8", 8"') angeordnet ist, so dass diese mit der zumindest einen Spule (18, 18') der Übertragungseinheit (12', 12", 12"') koppelbar ist.

8. Kupplungsvorrichtung (1', 1", 1"') nach Anspruch 4, **dadurch gekennzeichnet, dass** die zumindest eine Spule (18, 18') der Übertragungseinheit (12', 12", 12"') im Bereich der Vertiefung (9) für das Positionierungselement (8', 8", 8"') an einer oder mehreren Seitenwänden (24) und/ oder an der Basisfläche (16) der Vertiefung (9) angeordnet ist, so dass diese mit der zumindest einen Spule (17, 17') der Speichereinheit (11', 11", 11"') koppelbar ist.

9. Kupplungsvorrichtung (1"') nach einem der Ansprüche 4 bis 8, **dadurch gekennzeichnet, dass** die Spulen (17', 18') der Speichereinheit (11"') und der Übertragungseinheit (12"') von einem weichmagnetischen Material (25), insbesondere Ferrit, Nickel-Zink oder Mangan-Zink, zum Leiten und/ oder Ausrichten der Feldlinien des magnetischen Felds in Richtung der ersten oder zweiten Spule und/ oder nicht elektrisch leitfähigem Material (26), insbesondere Kunststoff, Silikon, Harz, Keramik, Klebstoff, Lack oder Glas, zur Vermeidung der Schwächung des magnetischen Felds durch selbsterzeugte und/ oder empfangene Fremdfelder umgeben sind.

10. Kupplungsvorrichtung (1, 1', 1", 1"', 1"") nach einem der vorherigen Ansprüche, **dadurch gekennzeichnet, dass** das Positionierungselement (8, 8', 8", 8"', 8"") ein Gehäuse (27) umfasst, welches zumindest teilweise, insbesondere in jenem Bereich, der die Speichereinheit (11, 11', 11", 11"', 11"") umgibt, ein magnetisch nicht leitendes und elektrisch nicht leitendes Material, insbesondere Kunststoff, Glas oder Keramik, umfasst.

11. Kupplungsvorrichtung (1) nach einem der vorherigen Ansprüche, **dadurch gekennzeichnet, dass** das Positionierungselement (8) federnd gelagert ist, so dass dieses im Wesentlichen parallel zur Drehachse (10) der Kupplungsvorrichtung (1) verschiebbar ist.

12. Kupplungsvorrichtung (1, 1', 1", 1'"', 1"") nach einem der vorherigen Ansprüche, **dadurch gekennzeichnet, dass** das Positionierungselement (8, 8', 8", 8"', 8"") lösbar von dem Kupplungselement (4, 5) der Kupplungsvorrichtung (1, 1', 1", 1"', 1"") ausgebildet ist.

13. Kupplungsvorrichtung (1"") nach einem der vorherigen Ansprüche, **dadurch gekennzeichnet, dass** das Positionierungselement (8"") und die Übertragungseinheit (12"") weitere elektrische Kontakte und/ oder Lese- und/ oder Schreibvorrichtungen (28, 29) zur leitungsgebundenen und/ oder drahtlosen Übertragung von Daten, Signalen und/ oder Energie von dem einen Kupplungselement (4, 5) zu dem anderen Kupplungselement (4, 5) aufweist.

14. Verwendung einer Kupplungsvorrichtung (1, 1', 1", 1"', 1"") nach einem der vorherigen Ansprüche zur lösbaren Verbindung zweier medizinischer, insbesondere dentaler, Vorrichtungen.

15. Verwendung nach Anspruch 14, **dadurch gekennzeichnet, dass** zumindest eine der medizinischen, insbesondere dentalen, Vorrichtungen als Reinigungs- und/ oder Pflegegerät, als Antriebseinheit (3), Versorgungsschlauch oder als Instrument (2), insbesondere als Instrument (2) mit einer Antriebsvorrichtung für ein Werkzeug, ausgebildet ist.

## Claims

1. A coupling device (1, 1', 1", 1"', 1"") for detachably connecting a medical, in particular dental, instrument (2) to a drive unit (3) or a supply hose for transfer of data and/or energy and optionally a driving movement and/or a working medium, in particular a fluid, between the drive unit (3) or the supply hose and the instrument (2), comprising a first and a second coupling element (4, 5), wherein one of the two coupling elements (4, 5) is designed as a coupling recess (6) into which a coupling protrusion (7) of the other coupling element (4, 5) can be inserted, and wherein a positioning element (8, 8', 8", 8"', 8"") is disposed on one of the two coupling elements (4, 5) which can be inserted into an indentation (9) on the other coupling element (4, 5) to position the two coupling elements (4, 5) in a defined angular position about their shared axis (10) relative to one another, **characterized in that** the positioning element (8, 8', 8", 8"', 8"") comprises a memory unit (11, 11', 11", 11"', 11"") for storage of data, preferably instrument-related data, and the indentation (9) comprises a transfer unit (12, 12', 12", 12"', 12""), wherein the memory unit (11, 11', 11", 11"', 11"") is arranged in the positioning element (8, 8', 8", 8"', 8"") such, that it can be inserted into the indentation (9) of the other coupling element (4, 5), so that data can be transferred from the memory unit (11, 11', 11", 11"', 11"") of the one coupling element (4, 5) to the other coupling element (4, 5) of the coupling device (1, 1', 1", 1"', 1"") by means of the transfer unit (12, 12', 12", 12"', 12"").

2. The coupling device (1, 1"") according to claim 1, **characterized in that**
the memory unit (11, 11"") comprises at least one electric contact (14) which can be connected to an electric contact (13) of the transfer unit (12, 12""), so that the data, in particular instrument-related data, can be transferred in a hardwired operation.

3. The coupling device (1, 1"") according to claim 2, **characterized in that**
an electric contact (14), in particular that of the memory unit (11, 11"") is disposed in an end surface (15) of the positioning element (8, 8""), and an electric contact (13), in particular that of the transfer unit (12, 12"") is disposed in a base surface (16) of the indentation (9).

4. The coupling device (1', 1", 1"') according to claim 1, **characterized in that**
the memory unit (11', 11", 11'") and the transfer unit (12', 12", 12'") each have one reading and/or writing device (17, 17'; 18, 18'), in particular a first coil and a second coil, for output and/or writing of the memory element (19) with instrument-related data in particular.

5. The coupling device (1"') according to claim 4, **characterized in that**
the at least one first coil (17') of the memory unit (11"') and a memory element (19) of the memory unit (11"') are disposed over one another or side by side in particular on a shared carrier circuit board (20) in the positioning element (8"').

6. The coupling device (1") according to claim 4 or 5, **characterized in that**
the at least one first coil (17') of the memory unit (11") and a memory element (19) are disposed in a shared housing (21) in the positioning element (8").

7. The coupling device (1', 1", 1"') according to claims 4 to 6, **characterized in that**
the at least one coil (17, 17') of the positioning element (8', 8", 8"') is disposed in or on a plurality of side walls (23) and/or on the end surface (15) of the positioning element (8', 8", 8"'), so that the coil can be coupled to the at least one coil (18, 18') of the transfer unit (12', 12", 12"').

8. The coupling device (1', 1", 1"') according to claim 4, **characterized in that**
the at least one coil (18, 18') of the transfer unit (12', 12", 12"') is disposed in the area of the indentation (9) for the positioning element (8', 8", 8"') on one or more side walls (24) and/or on the base surface (16) of the indentation (9), so that the coil can be coupled to the at least one coil (17, 17') of the memory unit (11', 11", 11"').

9. The coupling device (1"') according to claims 4 to 8, **characterized in that**
the coils (17', 18') of the memory unit (11"') and the transfer unit (12"') are surrounded by a soft magnetic material (25), in particular ferrite, nickel-zinc or manganese-zinc for conducting and/or aligning the field lines of the magnetic field in the direction of the first or second coil and/or electrically nonconductive material (26), in particular plastic, silicone, resin, ceramic, adhesive, lacquer or glass, to prevent weakening of the magnetic field due to self-generated and/or received foreign fields.

10. The coupling device (1, 1', 1", 1"', 1"") according to any one of the preceding claims, **characterized in that**
the positioning element (8, 8', 8", 8"', 8"") comprises a housing (27), which is made of a magnetically nonconductive and electrically nonconductive material, in particular plastic, glass or ceramic at least partially, in particular in the region surrounding the memory unit (11, 11', 11", 11"', 11"").

11. The coupling device (1) according to any one of the preceding claims, **characterized in that**
the positioning element (8) is mounted resiliently, so that it can be displaced essentially in parallel with the axis of rotation (10) of the coupling device (1).

12. The coupling device (1, 1', 1", 1"', 1"") according to any one of the preceding claims, **characterized in that**
the positioning element (8, 8', 8", 8"', 8"") is designed to be releasable from the coupling element (4, 5) of the coupling device (1, 1', 1", 1"', 1"").

13. The coupling device (1"") according to any one of the preceding claims, **characterized in that**
the positioning element (8"") and the transfer unit (12"") comprise additional electric contacts and/or reading and/or writing devices (28, 29) for hardwired and/or wireless transfer of data, signals and/or energy from the one coupling element (4, 5) to the other coupling element (4, 5).

14. Use of a coupling device (1, 1', 1", 1"', 1"") according to any one of the preceding claims for detachably connecting two medical, in particular dental, devices.

15. Use according to claim 14, **characterized in that**
at least one of the medical, in particular dental, devices is designed as a cleaning and/or care device, as a drive unit (3), a supply hose or as an instrument (2), in particular as an instrument (2) having a drive device for a tool.

## Revendications

1. Dispositif d'accouplement (1, 1', 1", 1"', 1"") pour la liaison amovible d'un instrument médical, en particulier dentaire (2), avec une unité d'entraînement (3) ou un tube d'alimentation pour la transmission de données et/ou d'énergie et le cas échéant d'un mouvement d'entraînement et/ou d'un milieu de travail, en particulier un fluide, entre l'unité d'entraînement (3) ou le tube d'alimentation et l'instrument (2), présentant un premier et un deuxième élément d'accouplement (4, 5), dans lequel l'un des deux éléments d'accouplement (4, 5) est réalisé en tant que cavité d'accouplement (6) dans laquelle une saillie d'accouplement (7) de l'autre élément d'accouplement (4, 5) peut être insérée, et dans lequel sur l'un des deux éléments d'accouplement (4, 5) est disposé un élément de positionnement (8, 8', 8", 8"', 8""), lequel peut être inséré dans un évidement (9) au niveau de l'autre élément d'accouplement (4, 5) afin de positionner l'un par rapport à l'autre les deux éléments d'accouplement (4, 5) dans une position angulaire définie autour de leur axe (10) commun, **caractérisé en ce que** l'élément de positionnement (8, 8', 8", 8"', 8"") présente une unité de mémoire (11, 11', 11", 11"', 11"") pour le stockage de données de préférence relatives à l'instrument et **en ce que** l'évidement (9) présente une unité de transmission (12, 12', 12", 12"', 12""), dans lequel l'unité de mémoire (11, 11', 11", 11"', 11"") est disposée dans l'élément de positionnement (8, 8', 8", 8"', 8"") d'une façon telle, que celle-ci peut être insérée dans l'évidement (9) de l'autre élément d'accouplement (4, 5) pour la transmission, au moyen de l'unité de transmission (12, 12', 12", 12"', 12""), des données de l'unité de mémoire (11, 11', 11", 11"', 11"") de l'un des éléments d'accouplement (4, 5) à l'autre élément d'accouplement (4, 5) du dispositif d'accouplement (1, 1', 1", 1"', 1"").

2. Dispositif d'accouplement (1, 1"") selon la revendication 1, **caractérisé en ce que** l'unité de mémoire (11, 11"") présente au moins un contact électrique (14) pouvant être raccordé à un contact électrique (13) de l'unité de transmission (12, 12"") de manière à ce que les données, en particulier relatives à l'instrument, puissent être transmises de façon filaire.

3. Dispositif d'accouplement (1, 1"") selon la revendication 2, **caractérisé en ce qu**'un contact électrique (14), en particulier celui de l'unité de mémoire (11, 11""), est disposé dans une surface frontale (15) de l'élément de positionnement (8, 8"') et en ce qu'un contact électrique (13), en particulier celui de l'unité de transmission (12, 12""), est disposé dans une surface de base (16) de l'évidement (9).

4. Dispositif d'accouplement (1', 1", 1"') selon la revendication 1, **caractérisé en ce que** l'unité de mémoire (11', 11", 11"') et l'unité de transmission (12', 12", 12"') comprennent respectivement un dispositif de lecture et/ou d'écriture (17, 17' ; 18, 18'), en particulier une première et une deuxième bobine, pour la lecture et/ou l'écriture de données, en particulier relatives à l'instrument, dans l'élément de mémoire (19).

5. Dispositif d'accouplement (1"') selon la revendication 4, **caractérisé en ce que** l'au moins une première bobine (17') de l'unité de mémoire (11"') et un élément de mémoire (19) de l'unité de mémoire (11"') sont disposés, en particulier en superposition ou côte à côte, sur une platine de support commune (20) dans l'élément de positionnement (8"').

6. Dispositif d'accouplement (1") selon la revendication 4 ou 5, **caractérisé en ce que** l'au moins une première bobine (17') de l'unité de mémoire (11") et l'élément de mémoire (19) sont disposés dans un boîtier commun (21) dans l'élément de positionnement (8").

7. Dispositif d'accouplement (1', 1", 1"') selon l'une des revendications 4 à 6, **caractérisé en ce que** l'au moins une bobine (17, 17') de l'élément de positionnement (8', 8", 8"') est disposée sur une ou sur plusieurs parois latérales (23) et/ou sur la surface frontale (15) de l'élément de positionnement (8', 8", 8"') de manière à ce que celle-ci puisse être accouplée à l'au moins une bobine (18, 18') de l'unité de transmission (12', 12", 12"').

8. Dispositif d'accouplement (1', 1", 1"') selon la revendication 4, **caractérisé en ce que** l'au moins une bobine (18, 18') de l'unité de transmission (12', 12", 12"') est disposée, dans la zone de l'évidement (9) pour l'élément de positionnement (8', 8", 8"'), sur une ou plusieurs parois latérales (24) et/ou sur la surface de base (16) de l'évidement (9), de manière à ce que celle-ci puisse être accouplée à l'au moins une bobine (17, 17') de l'unité de mémoire (11', 11", 11"').

9. Dispositif d'accouplement (1"') selon l'une des revendications 4 à 8, **caractérisé en ce que** les bobines (17', 18') de l'unité de mémoire (11"') et de l'unité de transmission (12"') sont entourées par un matériau magnétique doux (25), en particulier de la ferrite, du nickel-zinc ou du manganèse-zinc, pour guider et/ou orienter les lignes de champ du champ magnétique en direction de la première ou deuxième bobine et/ou par un matériau non électroconducteur (26), en particulier du plastique, du silicone, de la résine, de la céramique, de la colle, du vernis ou du verre, pour éviter l'affaiblissement du champ magnétique par des champs étrangers auto-générés et/ou réceptionnés.

10. Dispositif d'accouplement (1, 1', 1", 1"', 1"") selon l'une des revendications précédentes, **caractérisé en ce que** l'élément de positionnement (8, 8', 8", 8"', 8"") comprend un boîtier (27) comprenant au moins partiellement, en particulier dans la zone qui entoure l'unité de mémoire (11, 11', 11", 11"', 11""), un matériau non conducteur magnétiquement et non électroconducteur, en particulier du plastique, du verre ou de la céramique.

11. Dispositif d'accouplement (1) selon l'une des revendications précédentes, **caractérisé en ce que** l'élément de positionnement (8) est monté de façon élastique de manière à ce que celui-ci puisse coulisser sensiblement parallèlement à l'axe de rotation (10) du dispositif d'accouplement (1).

12. Dispositif d'accouplement (1, 1', 1", 1"', 1"") selon l'une des revendications précédentes, **caractérisé en ce que** l'élément de positionnement (8, 8', 8", 8"', 8"") est réalisé de manière détachable de l'élément d'accouplement (4, 5) du dispositif d'accouplement (1, 1', 1", 1"', 1"").

13. Dispositif d'accouplement (1"") selon l'une des revendications précédentes, **caractérisé en ce que** l'élément de positionnement (8"") et l'unité de transmission (12"") présentent d'autres contacts électriques et/ou dispositifs de lecture et/ou d'écriture (28, 29) pour la transmission filaire et/ou sans fil de données, signaux et/ou d'énergie de l'un des éléments d'accouplement (4, 5) vers l'autre élément d'accouplement (4, 5).

14. Utilisation d'un dispositif d'accouplement (1, 1', 1", 1"', 1"") selon l'une des revendications précédentes pour la liaison amovible de deux dispositifs médicaux, en particulier dentaires.

15. Utilisation selon la revendication 14, **caractérisée en ce qu**'au moins l'un des dispositifs médicaux, en particulier dentaires, est réalisé en tant qu'appareil de nettoyage et/ou de soins, en tant qu'unité d'entraînement (3), tube d'alimentation ou en tant qu'instrument (2), en particulier en tant qu'instrument (2) avec un dispositif d'entraînement pour un outil.
